(19) Europäisches Patentamt
European Patent Office
Office européen des brevets

(11) Publication number: **0 166 302**
**A2**

(12) **EUROPEAN PATENT APPLICATION**

(21) Application number: **85107220.7**

(22) Date of filing: **12.06.85**

(51) Int. Cl.4: **C 07 D 243/08**, A 61 K 31/55

(30) Priority: **19.06.84 IT 2147184**

(43) Date of publication of application: **02.01.86 Bulletin 86/1**

(84) Designated Contracting States: **AT BE CH DE FR GB IT LI LU NL SE**

(71) Applicant: **POLI INDUSTRIA CHIMICA S.p.A., Piazza Agrippa, 1, I-20141 Milano (IT)**

(72) Inventor: **Massaroli, Gian Giacomo, Via Lauro, Rovegnate (Como) (IT)**

(74) Representative: **Bianchetti, Giuseppe, Studio Consulenza Brevettuale Via Senato 24, I-20121 Milan (IT)**

(54) **"1-Bis-(4-fluorophyl)methyll-4-(3-phenyl-2-propenyl)-hexahydro-1H-1,4-diazepine, preparation thereof and compositions containing it.**

(57) The compound 1-[bis-(4-fluorophenyl)methyl]-4-(3-phenyl-2-propenyl)-hexahydro-1H-1,4-diazepine of formula I

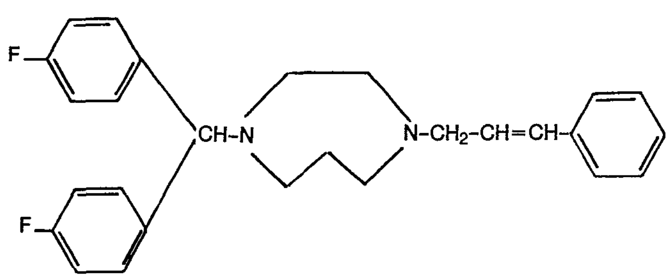

(I)

is endowed with calcium antagonistic activity useful in human therapy.

# 1-[BIS-(4-FLUOROPHENYL)METHYL]-4-(3-PHENYL-2-PROPENYL)-HEXAHYDRO-1H-1,4-DIAZEPINE, PREPARATION THEREOF AND COMPOSITIONS CONTAINING IT

The present invention refers to 1-[bis-(4-fluorophenyl)methyl]-4-(3-phenyl-2-propenyl)-hexahydro-1H-1,4-diazepine of formula I

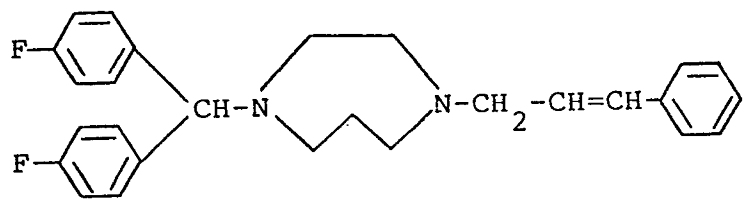

(I)

and its non toxic, pharmaceutically acceptable salts.

A particularly preferred salt is the dihydrochloride of compound I, hereinafter specifically referred to in the disclosure.

A further object of the invention is provided by a process for the preparation of compound I and of its salts and by pharmaceutical compositions containing them as active principle.

The compound I is endowed with calcium antagonistic activity, which has been determined in vitro on fragments of rat's thoracic aorta depolarized by $K^+$, according to its ability to inhibit the $Ca^{++}$ induced spasms.

The compound I proved to be highly active in reducing "in vitro" the increase of the intraluminal pressure of the rat's caudal artery, induced by electrical stimuli.

In this test the concentration of compound I which causes a 50% increase of the intraluminal pressure turned

out to be comprised between 1 and 2 mcg/ml.

"In vivo", the compound according to the present invention proved to be remarkably active in decreasing the increase of the peripheric vascular resistances of the cat's rear limb, induced by continuous infusion or noradrenaline.

The dose of I reducing of 50% the peripheric vascular resistances turned out to be comprised in this test, between 50 mg/kg and 75 mg/kg by endovenous route.

As a consequence, the compound I is useful in human therapy for the treatment of circulatory diseases of different kind and nature, being moreover endowed with a low toxicity.

The compound I may be administered in form of pharmaceutical compositions containing it or one of its salt as the active principle, alone or in combination with the conventional excipients, carriers, sweetening and flavouring agents normally used in the pharmaceutical practice.

The process for the preparation of the compound I, according to the present invention, is illustrated in the following scheme:

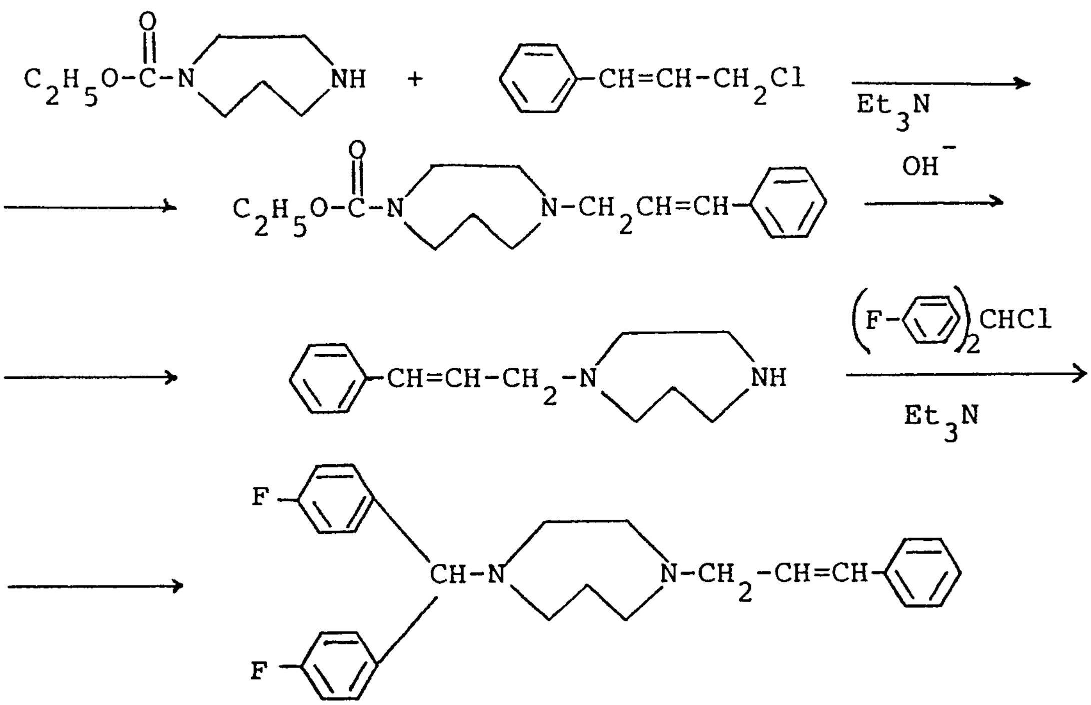


The 1-ethoxycarbonyl-hexahydro-1,4-diazepine is alkylated by a cynnamyl halide in the presence of an acidity acceptors: the ethoxycarbonyl group is then removed by alkaline hydrolysis and the resulting compound is reacted with a 4,4'-difluoro-benzhydril halide, in the presence of bases. The compound so obtained may be then optionally salified according to known methods.

Suitable solvents which can be suitably used are acetone, ethyl acetate, methylisobutylketone, methylene chloride etc.

It is moreover obvious that protective groups other than the ethoxycarbonyl one may be used.

The following example further illustrates the invention without limiting it in any way.

EXAMPLE

a) 4-(3-Phenyl-2-propenyl)-1H-hexahydro-1,4-diazepine

39.65 Grams (0.26 moles) of cinnamyl chloride and 39.2 g (0.4 moles) of triethylamine were added to 34.4 g

(0.2 moles) of 1-ethoxycarbonyl-hexahydro-1,4-diazepine in 200 ml of methylisobutylketone. The mixture was refluxed for 3 hours, cooled and poured into water. The organic phase was separated, washed with water, the solvent was evaporated and the residue distilled under reduced pressure, obtaining 51 g (88.5% yield) of a product having b.p. 183-186°/$_{0.2\ mm}$ which was refluxed for 20 hours with 1000 ml of ethanol containing 200 g of KOH, obtaining after partial evaporation of the solvent, dilution with water, extraction with toluene and distillation 25.7 g of 4-(3-phenyl-2-propenyl)-1H-hexahydro-1,4-diazepine having b.p. 142-144°/$_{0.2\ mm}$. Yield 59.3%.

b) **1-[Bis-(4-fluorophenyl)methyl]-4-(3-phenyl-2-propenyl)-hexahydro-1H-1,4-diazepine, dihydrochloride**

A solution of 10.8 g (0.05 moles) of 4-(3-phenyl-2-propenyl)-1H-hexahydro-1,4-diazepine, 11.92 g (0.05 moles) of bis(4-fluorophenyl)-chloromethane and 14 ml (0.1 ml) of triethylamine in 50 ml of methylisobutylketone was refluxed for 6 hours. After cooling, the mixture was washed with water to neutrality, the organic phase was preparated and dried on anhydrous sodium sulphate and treated under stirring with 0.1 ml of gaseous hydrochloric acid.

The dihydrochloride was filtered and crystallized from absolute ethanol, obtaining 18 g (73.2% yield) of 1-[bis-(4-fluorophenyl)methyl]-4-(3-phenyl-2-propenyl)-hexahydro-1H-1,4-diazepine, dihydrochloride having melting point 175°C.

Argentometric assay: 99.7%.

Acidimetric assay (perchloric acid): 99.9%.

The F, N, C, H values are in agreement with the

reported formula I.

The compound so prepared can be administered by the oral route in form of tablets, sugar-coated tablets, capsules, drops, solution and the like.

The following formulations are cited by way of an example:

a) **tablets** containing from 5 to 25 mg of compound I, mixed with excipients and disgregants conventionally used in the pharmaceutical practice;

b) **solution** for the administration in form of drops, containing from 5 to 25 mg of compound I in 1 ml of hydroalcoholic vehicle containing the conventional sweetening and flavouring agents commonly used in the pharmaceutical technique.

CLAIMS for the Contracting States:

BE, CH, DE, FR, GB, IT, LI, LU, NL, SE

1. 1-/Bis-(4-fluorophenyl)methyl7-4-(3-phenyl-2-propenyl)-hexahydro-1H-1,4-diazepine of formula I

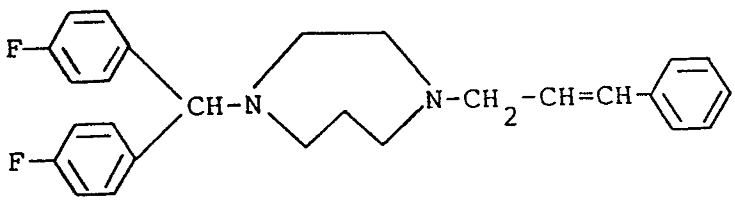

(I)

and salts thereof with non toxic, pharmaceutically acceptable acids.

2. 1-/Bis-(4-fluorophenyl)methyl7-4-(3-phenyl-2-propenyl)-hexahydro-1H-1,4-diazepine dihydrochloride.

3. A process for the preparation of the compound of formula I characterized in that 1-ethoxycarbonyl-hexahydro-1,4-diazepine is reacted with a cinnamyl halide in the presence of bases, that the ethoxycarbonyl group is removed by alkaline hydrolysis and that the obtained compounds is reacted with a 4,4'-difluoro-benzhydryl halide, in the presence of bases.

4. A process according to claim 3 characterized by using cinnamyl chloride and bis-(4-fluorophenyl)-chloromethane in the presence of triethylamine.

5. Pharmaceutical compositions having calcium antagonistic activity containing as the active principle the compound I or one of its salts in admixture with suitable vehicles and excipients.

6. Compositions according to claim 4 characterized by

containing as active principle the dihydrochloride of compound I.

7. Oral, liquid or solid compositions containing from 5 to 25 mg of the compound I or of salts thereof.

CLAIMS for AT

1. A process for the preparation of the compound of formula I

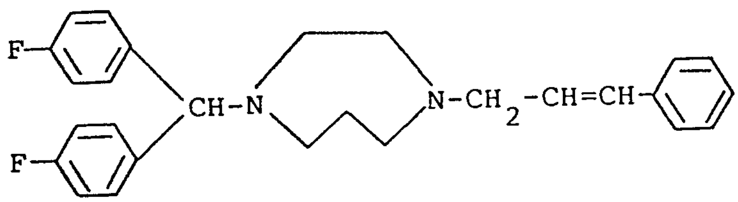

(I)

characterized in that 1-ethoxycarbonyl-hexahydro-1,4-diazepine is reacted with a cinnamyl halide in the presence of bases, that the ethoxycarbonyl group is removed by alkaline hydrolysis and that the obtained compounds is reacted with a 4,4'-difluoro-benzhydryl halide, in the presence of bases.

2. A process according to claim 1 characterized by using cinnamyl chloride and bis-(4-fluorophenyl)-chloromethane in the presence of triethylamine.